(19) Europäisches Patentamt
European Patent Office
Office européen des brevets

(11) Veröffentlichungsnummer: **0 075 140**
**B1**

(12) # EUROPÄISCHE PATENTSCHRIFT

(45) Veröffentlichungstag der Patentschrift: **19.12.84**

(51) Int. Cl.³: **C 07 D277/60**, C 07 D277/82, C 07 D277/84, A 61 K 31/425

(21) Anmeldenummer: **82107952.2**

(22) Anmeldetag: **30.08.82**

(54) **Tricyclische Thiazolyloxamidsäuren und Derivate, Verfahren zu ihrer Herstellung und diese enthaltende therapeutische Mittel.**

(30) Priorität: **05.09.81 DE 3135250**

(43) Veröffentlichungstag der Anmeldung: **30.03.83 Patentblatt 83/13**

(45) Bekanntmachung des Hinweises auf die Patenterteilung: **19.12.84 Patentblatt 84/51**

(84) Benannte Vertragsstaaten: **AT BE CH DE FR GB IT LI NL SE**

(56) Entgegenhaltungen:
**DE-A- 2 509 457**
**DE-A- 2 656 468**
**DE-A- 2 751 441**
**Chemical Abstracts Band 78, Nr. 23, 11. Juni 1973, Columbus, Ohio, USA Seite 382, Spalte 2, Abstract Nr. 147943d**
**Chemical Abstracts Band 45, Nr. 7, 10 April 1951 Columbus, Ohio, USA L.C. KING et al. "Reaction of ketones with iodine and thiourea", Spalte 2934**

(73) Patentinhaber: **BASF Aktiengesellschaft**
**Carl-Bosch-Strasse 38**
**D-6700 Ludwigshafen (DE)**

(72) Erfinder: **Schlecker, Rainer, Dr.**
**Suedring 8**
**D-6719 Bissersheim (DE)**
Erfinder: **Friedrich, Ludwig, Dr.**
**Nibelungenstrasse 8A**
**D-6831 Bruehl (DE)**
Erfinder: **Lenke, Dieter, Dr.**
**Kekuleplatz 1**
**D-6700 Ludwigshafen (DE)**

**EP 0 075 140 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Benzocycloalkanothiazolyloxamidsäuren und ihre Ester und Salze, Verfahren zu ihrer Herstellung und diese enthaltende pharmazeutische Zubereitungen, die als wertvolle Arzneimittel bei der Behandlung von allergischen Erkrankungen verwendet werden können.

Es sind eine Reihe von Derivaten der Oxamidsäure und ihrer Ester bekannt, beispielsweise aus der DE-A-24 13 966 Aryl- und Hetarylderivate, der DE-A-28 28 091 und der veröffentlichten europäischen Patentanmeldung EP-A-00 06 368 Thiazolderivate und aus der DE-A-27 51 441 und 26 56 468 Benzothiazolderivate, für die eine Verwendung bei der Bekämpfung oder Unterdrückung von allergischen Reaktionen beschrieben wird. Ihre Wirkungen befriedigen jedoch nicht immer. Weitere, heterocyclisch substituierte Oxaminderivate mit antiallergischer Wirkung sind in der DE-A-25 09 457 offenbart.

Es wurde gefunden, daß Verbindungen der Formel I

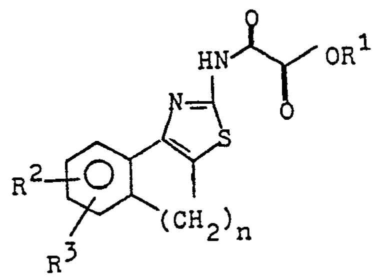

(I)

in der

n eine ganze Zahl von 1 bis 6, wobei gegebenenfalls ein Wasserstoffatom in der $-(CH_2)_n$-Gruppe durch eine niedere Alkylgruppe mit 1 bis 6 Kohlenstoffatomen oder einen Phenylring oder zwei geminale Wasserstoffatome durch ein Sauerstoffatom ersetzt sein können.

$R^1$ ein Wasserstoffatom, gegebenenfalls ersetzt durch ein physiologisch verträgliches Metall- oder Aminkation, oder einen Alkylrest mit 1 bis 6 Kohlenstoffatomen,

$R^2$ und $R^3$, die gleich oder verschieden sein können, ein Wasserstoff-, Fluor-, Chlor-, Brom- oder Iodatom, eine Nitrogruppe, eine Amino-, Alkylamino-, Dialkylamino-, Acylamino-, Alkoxy-, Alkylthio-, Alkylsulfenyl- oder Alkylsulfonylgruppe mit jeweils 1 bis 6 Kohlenstoffatomen im Alkyl, oder einen Hydroxy-, Acyl-, Cyano- oder Carboxylrest oder eine Alkylgruppe mit 1 bis 5 Kohlenstoffatomen bedeuten, wertvolle pharmakologische Eigenschaften, insbesondere als Antiallergika, aufweisen.

Von den Verbindungen der Formel I sind die bevorzugt, in denen

n eine ganze Zahl von 1 bis 4, wobei gegebenenfalls ein Wasserstoffatom in der $-(CH_2)_n$-Gruppe durch eine niedere Alkylgruppe mit 1 bis 6 Kohlenstoffatomen oder einen Phenylring oder zwei geminale Wasserstoffatome durch ein Sauerstoffatom ersetzt sein können,

$R^1$ ein Wasserstoffatom, gegebenenfalls ersetzt durch ein physiologisch verträgliches Metall- oder Aminkation, oder einen Alkylrest von 1 bis 6 Kohlenstoffatomen,

$R^2$ und $R^3$, die gleich oder verschieden sein können, ein Wasserstoff- oder Chloratom, eine Alkyl- oder Alkoxygruppe mit 1 bis 4 Kohlenstoffatomen bedeuten.

Besonders bevorzugt sind Verbindungen der allgemeinen Formel I, in denen n eine ganze Zahl von 1 bis 3, wobei gegebenenfalls zwei geminale Wasserstoffatome durch einen Sauerstoff ersetzt sein können, $R^1$ ein Wasserstoffatom, gegebenenfalls ersetzt durch ein physiologisch verträgliches Metall- oder Aminkation, oder einen niederen Alkylrest mit 1 bis 4 Kohlenstoffatomen und $R^2$ und $R^3$ Wasserstoffatome darstellen.

Die Herstellung der Verbindungen der allgemeinen Formel I erfolgt, indem man in an sich bekannter Weise ein Aminothiazol der Formel II

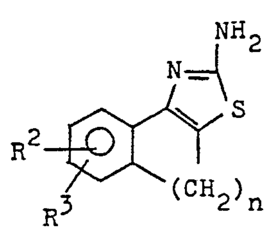

(II)

in der n, $R^2$, $R^3$ und $R^4$ die oben genannten Bedeutungen haben, mit einem Alkyloxalylhalogenid, wie Ethyloxalylchlorid, oder einem Dialkyloxalat, vorzugsweise Diethyloxalat, kondensiert und gegebenenfalls die erhaltene Verbindung zur Säure verseift und mit einem physiologisch verträglichen Metall- oder Aminkation in das Salz überführt.

Bei Verwendung eines Alkyloxalylhalogenides erfolgt die Reaktion zweckmäßig bei Temperaturen

von − 30 bis 50 °C, üblicherweise bei Raumtemperatur in einem inerten Lösungsmittel. Geeignete Lösungsmittel sind beispielsweise halogenierte Kohlenwasserstoffe, wie Methylenchlorid oder Chloroform, N-Dialkylcarboxamide, wie Dimethylformamid, cyclisch gesättigte Ether, wie Tetrahydrofuran oder Dioxan, und aromatische Kohlenwasserstoffe, wie Toluol. Die Umsetzung wird bevorzugt in Anwesenheit von Basen, wie Triethylamin oder Pyridin, durchgeführt.

Die Umsetzung von einer Verbindung der Formel II mit einem Dialkyloxalat kann mit oder ohne Zusatz eines Lösungsmittels, wie Toluol, Chlorbenzol oder Diphenylether, bei einer Temperatur zwischen Raumtemperatur und der Rückflußtemperatur der Reaktionsmischung, durchgeführt werden.

Die Alkyloxamate der Formel I können auch nach bekannten Verfahren, wie sie beispielsweise in Houben-Weyl, Bd. *8*, S. 526 bis 528 beschrieben sind, durch Umesterung aus Estern niedrigsiedender Alkohole erhalten werden.

Die Oxamidsäuren der Formel I ($R^1$ = H) werden durch saure oder alkalische Hydrolyse der Oxamidsäureester in einer wäßrigen oder wäßrig/alkoholischen Lösung hergestellt. Als Basen können beispielsweise Natriumhydroxid, Kaliumcarbonat oder Natriumcarbonat verwendet werden, als Säuren Salzsäure, Schwefel- oder Phosphorsäure. Die so erhaltenen organischen Säuren werden gegebenenfalls in ihr physiologisch verträgliches Amin- oder Metallsalz überführt. Darunter versteht man beispielsweise Salze der Alkalimetalle, wie Natrium und Kalium, der Erdalkalimetalle, wie Magnesium und Calcium, sonstiger Metalle, wie Aluminium, sowie Salze des Ammoniums oder von organischen Basen, wie Morpholin, Piperidin, Mono-, Di- und Triethanolamin oder Tris(hydroxymethyl)aminomethan.

Die Ausgangsverbindungen der Formel II sind zum großen Teil literaturbekannt oder können nach bekannten Verfahren, wie sie z. B. in « The Chemistry of Heterocyclic Compounds, Thiazoles and its Derivatives » Ed. J. V. Metzger, Bd. 1, S. 213 ff zusammengestellt sind, synthetisiert werden.

Neben den in den Ausführungsbeispielen beschriebenen Verbindungen können als erfindungsgemäße Verbindungen beispielsweise genannt werden :

N-(5-Methyl-8-oxo-indeno [1,2-d] thiazol-2-yl)oxamidsäure und ihr Methyl- und Ethylester,
N-(5-Methoxy-indeno [1,2-d] thiazol-2-yl)oxamidsäure und ihr Methyl-, Ethyl- und Isoamylester,
N-(8-Methyl-indeno [1,2-d] thiazol-2-yl)oxamidsäure und ihr Ethylester,
N-(6-Acetylamino-indeno [1,2-d] thiazol-2-yl)oxamidsäure und ihr Ethyl-, Propyl- und Butylester,
N-(7-Amino-indeno [1,2-d] thiazol-2-yl)oxamidsäure und ihr Ethyl- und Propylester,
N-(7-Methylthio-indeno [1,2-d]-thiazol-2-yl)oxamidsäure und ihr Methyl-, Ethyl-, Propyl- und Hexylester,
N-(6-Methylsulfenyl-indeno [1,2-d] thiazol-2-yl)-oxamidsäure und ihr Methyl- und Ethylester,
N-(5-Cyano-indeno [1,2-d] thiazol-2-yl)oxamidsäure und ihr Methyl- und Ethylester,
N-(7-Methoxy-benzo [5,6] cyclohexano [1,2-d] thiazol-2-yl)-oxamidsäure und ihr Methyl-, Ethyl- und Butylester,
N-(6-Methoxy-benzo [5,6] cyclohexano [1,2-d] thiazol-2-yl)-oxamidsäure und ihr Methyl-, Ethyl- und Isoamylester,
N-(5-Methoxy-benzo [5,6] cyclohexano [1,2-d] thiazol-2-yl)-oxamidsäure und ihr Methyl-, Ethyl- und Propylester,
N-(8-Methyl-benzo [5,6] cyclohexano [1,2-d] thiazol-2-yl)-oxamidsäure und ihr Methyl-, Ethyl- und Pentylester,
N-(6-Chlorbenzo [5,6] cyclohexano [1,2-d] thiazol-2-yl)oxamidsäure und ihr Methyl-, Ethyl- und Isoamylester,
N-(7-Nitro-benzo [5,6] cyclohexanon [1,2-d] thiazol-2-yl)oxamidsäure und ihr Methyl-, Ethyl- und Propylester,
N-(Benzo [6,7] cycloheptano [1,2-d] thiazol-2-yl)oxamidsäure und ihr Methyl-, Ethyl-, Propyl- und Isoamylester,
N-(5,6-Dihydroxy-benzo [6,7] cycloheptano [1,2-d] thiazol-2-yl)-oxamidsäure und ihr Methyl- und Ethylester,
N-(8-Phenyl-benzo [6,7] cycloheptano [1,2-d] thiazol-2-yl)-oxamidsäure und ihr Methyl-, Ethyl- und Hexylester,
N-(9-Methyl-benzo [6,7] cycloheptano [1,2-d] thiazol-2-yl)-oxamidsäure und ihr Methyl-, Ethyl- und Butylester,
N-(8-Oxo-benzo [6,7] cycloheptano [1,2-d] thiazol-2-yl)oxamidsäure und ihr Methyl-, Ethyl-, Propyl- und Butylester,
N-(Benzo [7,8] cyclooctano [1,2-d] thiazol-2-yl)oxamidsäure und ihr Methyl-, Ethyl-, Propyl- und Hexylester,
N-(7-Methyl-benzo [7,8] cyclooctano [1,2-d] thiazol-2-yl)oxamidsäure und ihr Methyl-, Ethyl- und Isoamylester,
N-(Benzo [8,9] cyclononano [1,2-d] thiazol-2-yl)oxamidsäure und ihr Methyl-, Ethyl-, Propyl- und Butylester,
N-(4-Methyl-benzo [8,9] cyclononano [1,2-d] thiazol-2-yl)-oxamidsäure und ihr Methyl-, Ethyl-, Propyl-, Butyl- und Isoamylester,
N-(Benzo [9,10] cyclodecano [1,2-d] thiazol-2-yl)oxamidsäure und ihr Methyl- und Ethylester,

N-(7-Chlor-benzo [9,10] cyclodecano [1,2-d] thiazol-2-yl)-oxamidsäure und ihr Methyl-, Ethyl- und Isoamylester.

Die erfindungsgemäßen Verbindungen weisen antiallergische Eigenschaften auf, aufgrund deren sie als wertvolle Heilmittel bei der Behandlung von allergischen Erkrankungen des Respirations-, des Gastro-Intestinaltraktes und der Haut, z. B. bei allergischer Asthma, allergischer Rhinitis oder Nahrungsmittel-Allergien, verwendet werden können. Gegenüber dem bekannten Antiallergikum Cromolyne zeichnen sich die erfindungsgemäßen Verbindungen im Tierexperiment bei der passiven cutanen Anaphylaxie der Ratte dadurch aus, daß sie oral wirksam sind.

Die antiallergische Wirkung wirde an Ratten getestet. Zur Prüfung wurde das Modell der passiven cutanen Anaphylaxie (PCA) verwendet.

Narkotisierte männliche Ratten (100 bis 140 g) werden durch intradermale Injektion (rasierte Rattenhaut) von 0,1 ml eines Ovalbumin-Antiserums sensibilisiert. Nach einer Sensibilisierungsperiode von ca. 48 Stunden erfolgt die Behandlung (intraperitoneale oder orale Applikation) mit unterschiedlichen Dosierungen (10 Tiere/Dosis) der Prüfsubstanzen. 15 bzw. 20 Minuten nach der Behandlung wird eine Antigen/Evansblau-Mischung (10 mg/kg Ovalbumin in 2 %iger Evansblau-Lösung) den Versuchstieren intravenös injiziert. 30 Minuten später werden die Tiere getötet, die Rückenhaut abgezogen und auf der inneren Oberfläche der Durchmesser der kreisförmigen Blaufärbung gemessen. Die Größe der Farbflecke unbehandelter Kontrolltiere ist standardisierbar. Antiallergisch wirksame Substanzen verkleinern dosisabhängig den Durchmesser der Farbflecke. Als ED 50 % wird die Dosis angegeben, die im Vergleich zu medikamentös unbehandelten Kontrolltieren den Durchmesser der Farbflecke um 50 % herabsetzt.

Die erfindungsgemäßen Verbindungen sind antiallergisch hoch wirksam. Aus Tabelle 1 geht hervor, daß diese Verbindungen nach oraler Gabe 4,4 (Beispiel 2) bis 6,6 (Beispiel 4) mal wirksamer sind als die Vergleichsverbindung $V_1$ (N-Benzthiazol-2-yl-oxamidsäureester, DE-OS 27 51 441 ; Beispiel 1). Das im Handel befindliche anerkannte Präparat Cromolyne (Intal[R]) ist bis 100 mg/kg per os unwirksam.

Tabelle 1

Antiallergische Wirkung nach oraler Gabe. PCA, Ratte

| Beispiel | Hemmung der PCA ED 50 % mg/kg | R.W. |
|---|---|---|
| 2 | 1,95 | 4,41 |
| 1 | 1,48 | 5,81 |
| 4 | 1,30 | 6,61 |
| $V_1$ 1) | 8,60 | 1,00 |
| Cromolyne | >100 | - |

1) N-Benzthiazol-2-yl-oxamidsäureester

Für die therapeutische Anwendung werden Einzeldosen von 0,1 bis 100 mg verwendet.

Gegenstand der vorliegenden Erfindung sind demnach auch therapeutische Mittel oder Zubereitungen, die neben pharmazeutisch üblichen Träger- und Verdünnungsmitteln eine Verbindung der Formel I als Wirkstoff enthalten, sowie die Verwendung der neuen Verbindungen bei der Behandlung von allergischen Erkrankungen.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Weiterhin kommen Inhalate und parenterale Zubereitungen, wie Injektionslösungen, in Betracht.

Die galenischen Applikationsformen, fest oder flüssig, werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln, verarbeitet werden (vgl. L. G. Goodman, A. Gilman, The Pharmacological Basis of Therapeutics).

Beispiel 1

N-(Indeno [1,2-d] thiazol-2-yl)oxamidsäureethylester

Eine Lösung von 5,2 g (0,028 Mol) 2-Amino-indeno [1,2-d]-thiazol in 250 ml Methylenchlorid/2,5 ml Pyridin wird bei Raumtemperatur tropfenweise mit 4,2 g (0,031 Mol) Oxalsäuremonoethylesterchlorid versetzt und über Nacht gerührt. Die Reaktionsmischung wird mit Wasser gewaschen über $Na_2SO_4$ getrocknet, das Lösungsmittel abdestilliert und der Rückstand aus Aceton umkristallisiert. 5,0 g (62 %), Fp. 226-228 °C.

$C_{14}H_{13}N_2O_3S$ (289)

Ber. 58,1 C 4,5 H 9,7 N
Gef. 58,4 C 4,5 H 9,8 N

In gleicher Weise werden ausgehend von den entsprechenden 2-Aminothiazolen erhalten:

Beispiel 2

N-(8-Oxo-indeno [1,2-d] thiazol-2-yl)oxamidsäureethylester

Ausb. 53 %, Fp. 237-238 °C (Ethylacetat)

$C_{14}H_{10}N_2SO_4$ (302)

Ber. 55,6 C 3,3 H 9,3 N
Gef. 56,0 C 3,4 H 9,2 N

Beispiel 3

N-(8-Phenyl-indeno [1,2-d] thiazol-2-yl)oxamidsäureethylester

Ausb. 16 %, Fp. 177-179 °C

$C_{20}H_{16}N_2O_3S$ (364)

Ber. 65,9 C 4,4 H 7,7 N
Gef. 65,5 C 4,6 H 7,6 N

Beispiel 4

N-(Indenol [1,2-d] thiazol-2-yl)oxamidsäure

3,5 g (0,012 Mol) N-(Indeno [1,2-d] thiazol-2-yl)oxamidsäureethylester werden in einer Lösung von 1,1 g (0,013 Mol) $NaHCO_3$ in 200 ml Wasser suspendiert und 4 Stunden unter Rückfluß erhitzt. Die Reaktionsmischung wird filtriert und das Filtrat mit verd. HCl auf pH 1 gestellt. Der Niederschlag wird abgesaugt und getrocknet. 2,9 g (93 %), Fp. 229-231 °C.

$C_{12}H_8N_2O_3S$

Ber. 55,4 C 3,1 H 10,8 N
Gef. 55,5 C 3,4 H 11,1 N

Beispiele für pharmazeutische Zubereitungen

Tabletten

| | |
|---|---|
| a) Wirkstoff | 0,100 g |
| Stearinsäure | 0,010 g |
| Traubenzucker | 1,890 g |
| | 2,000 g |

| | |
|---|---|
| b) Wirkstoff | 0,020 g |
| Starinsäure | 0,20 g |
| Traubenzucker | 1,960 g |
| | 2,000 g |

Die Bestandteile werden in üblicher Weise zu Tabletten der vorstehend angegebenen Zusammensetzung verarbeitet.

Inhalationsaerosol

| | |
|---|---|
| Wirkstoff | 1,00 Gew.-Teile |
| Sojalecithin | 0,20 Gew.-Teile |
| Treibgasmischung (Frigen 11, 12 und 114) ad | 100,00 Gew.-Teile |

Die Zubereitung wird vorzugsweise in Aerosolbehälter mit Dosierventil abgefüllt. Der einzelne Hub wird so bemessen, daß eine Dosis von 5 bis 20 mg Wirkstoff angegeben wird.

Ampullen (Injektionslösungen)

| | |
|---|---|
| Wirkstoff | 50,0 Gew.-Teile |
| Natriumpyrosulfit | 1,0 Gew.-Teil |
| Dinatriumsalz der Ethylendiamintetraessigsäure | 0,5 Gew.-Teile |
| Natriumchlorid | 8,5 Gew.-Teile |
| doppelt destilliertes Wasser ad | 1 000,0 Gew.-Teile |

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml Ampullen abgefüollt. Zuletzt werden die Ampullen sterilisiert und verschlossen. Jede Ampulle enthält 50 mg Wirkstoff.

**Ansprüche**

1. Verbindungen der Formel I

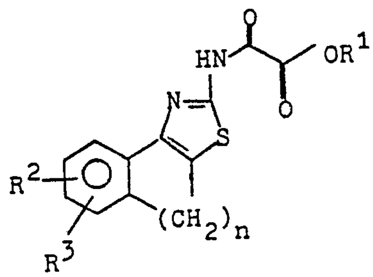

I

in der

n eine ganze Zahl von 1 bis 6, wobei gegebenenfalls ein Wasserstoffatom in der $-(CH_2)_n$-Gruppe durch eine niedere Alkylgruppe mit 1 bis 6 Kohlenstoffatomen oder einen Phenylring oder zwei geminale Wasserstoffatome durch ein Sauerstoffatom ersetzt sein können,

$R^1$ ein Wasserstoffatom, gegebenenfalls ersetzt durch ein physiologisch verträgliches Metall- oder Aminkation, oder einen Alkylrest mit 1 bis 6 Kohlenstoffatomen,

$R^2$ und $R^3$, die gleich oder verschieden sein können, ein Wasserstoff-, Fluor-, Chlor-, Brom- oder Iodatom, eine Nitrogruppe, eine Amino-, Alkylamino-, Dialkylamino-, Acylamino-, Alkoxy-, Alkylthio-, Alkylsulfenyl- oder Alkylsulfonylgruppe mit jeweils 1 bis 6 Kohlenstoffatomen im Alkyl oder einen Hydroxy-, Acyl-, Cyano- oder Carboxylrest oder eine Alkylgruppe mit 1 bis 5 Kohlenstoffatomen bedeuten.

2. Verbindungen der Formel I nach Anspruch 1, in denen

n eine ganze Zahl von 1 bis 4, wobei gegebenenfalls ein Wasserstoffatom in der $-(CH_2)_n$-Gruppe durch eine niedere Alkylgruppe mit 1 bis 6 Kohlenstoffatomen oder einen Phenylring oder zwei geminale Wasserstoffatome durch ein Sauerstoffatom ersetzt sein können,

$R^1$ ein Wasserstoffatom, gegebenenfalls ersetzt durch ein physiologisch verträgliches Metall- oder Aminkation, oder einen Alkylrest von 1 bis 6 Kohlenstoffatomen,

$R^2$ und $R^3$, die gleich oder verschieden sein können, ein Wasserstoff- oder Chloratom, eine Alkyl- oder Alkoxygruppe mit 1 bis 4 Kohlenstoffatomen bedeuten.

3. N-(Indeno [1,2-d] thiazol-2-yl)oxamidsäure, ihre Alkylester mit 1 bis 4 Kohlenstoffatomen im Alkyl und ihre Salze mit einem physiologisch verträglichen Metall- oder Aminkation.

4. Verfahren zur Herstellung von Verbindungen der Formel I nach Anspruch 1, dadurch gekennzeichnet, daß man in an sich bekannter Weise ein Aminothiazol der Formel II

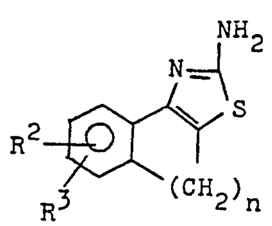

II

in der n, $R^2$, $R^3$ und $R^4$ die oben genannten Bedeutungen haben, mit einem Alkyloxalylhalogenid oder einem Dialkyloxalat kondensiert und gegebenenfalls die erhaltene Verbindung zur Säure verseift und mit einem physiologisch verträglichen Metall- oder Aminkation in das Salz überführt.

5. Therapeutisches Mittel, enthaltend eine Verbindung der Formel I nach Anspruch 1 als Wirkstoff neben pharmazeutisch üblichen Träger- und Verdünnungsmitteln.

6. Verbindung der Formel I nach Anspruch 1 als Arzneimittel bei der Bekämpfung von allergischen Erkrankungen.

**Claims**

1. A compound of the formula I

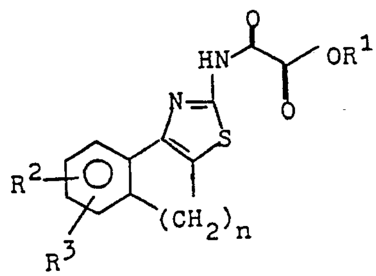

I

where

n is an integer from 1 to 6, and one hydrogen atom in the $-(CH_2)_n$-group may be replaced by lower alkyl of 1 to 6 carbon atoms or by phenyl, or two geminal hydrogen atoms may be replaced by one oxygen atom,

$R^1$ is hydrogen or a physiologically tolerated metal or amine cation, or is alkyl of 1 to 6 carbon atoms,

$R^2$ and $R^3$ are identical or different and each is hydrogen, fluorine, chlorine, bromine, iodine, nitro or amino, or alkylamino, dialkylamino, acylamino, alkoxy, alkylthio, alkylsulfenyl or alkylsulfonyl, where each alkyl is of 1 to 6 carbon atoms, or each is hydroxyl, acyl, cyano or carboxyl, or alkyl of 1 to 5 carbon atoms.

2. A compound of the formula I as claimed in claim 1, where

n is an integer from 1 to 4, and one hydrogen atom in the $-(CH_2)_n$-group may be replaced by lower alkyl of 1 to 6 carbon atoms or by phenyl, or two geminal hydrogen atoms may be replaced by one oxygen atom,

$R^1$ is hydrogen or a physiologically tolerated metal or amine cation, or is alkyl of 1 to 6 carbon atoms, and

$R^2$ and $R^3$ are identical or different and each is hydrogen or chlorine, or alkyl or alkoxy where each alkyl is of 1 to 4 carbon atoms.

3. N-(Indeno [1,2-d] thiazol-2-yl)-oxamic acid, its alkyl esters, where alkyl is of 1 to 4 carbon atoms, and its salts with a physiologically tolerated metal or amine cation.

4. A process for the preparation of a compound of the formula I as claimed in claim 1, wherein an amino-thiazole of the formula II

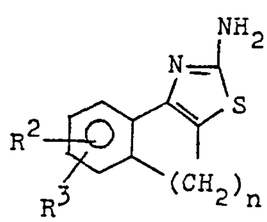

II

where n, $R^2$, $R^3$ and $R^4$ have the above meanings, is condensed in a conventional manner with an alkyloxalyl halide or with a dialkyl oxalate, and, if required, the resulting compound is hydrolyzed to give the acid, which is reacted with a physiologically tolerated metal or amine cation to give the salt.

5. A therapeutic agent containing a compound of the formula I as claimed in claim 1 as active ingredient in addition to usual pharmaceutical carriers and diluents.

6. A compound of the formula I as claimed in claim 1 as a drug for the treatment of allergic disorders.

**Revendications**

1. Composés de formule I

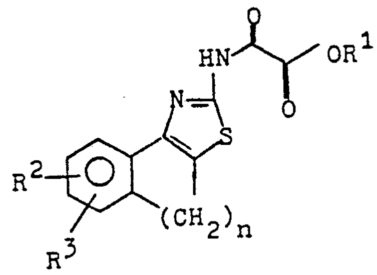

I

dans laquelle

n représente un nombre entier de 1 à 6, un atome d'hydrogène dans le groupe $-(CH_2)_n$ pouvant éventuellement être remplacé par un groupe alkyle inférieur ayant 1 à 6 atomes de carbone, ou par un noyau phényle, ou deux atomes d'hydrogène géminés pouvant être remplacés par un atome d'oxygène,

$R^1$ représente un atome d'hydrogène éventuellement remplacé par un cation métallique ou un cation amine physiologiquement tolérés, ou un reste alkyle ayant 1 à 6 atomes de carbone,

$R^2$ et $R^3$, qui peuvent être identiques ou différents, représentent un atome d'hydrogène, de fluor, chlore, brome ou iode, un groupe nitro, un groupe amino, alkylamino, dialkylamino, acylamino, alcoxy, alkylthio, alkylsulfényle ou alkylsulfonyle ayant chacun 1 à 6 atomes de carbone dans l'alkyle, ou un reste hydroxy, acyle, cyano ou carboxyle ou un groupe alkyle ayant 1 à 5 atomes de carbone.

2. Composés de formule I, selon la revendication 1, dans lesquels

n représente un nombre entier de 1 à 4, un atome d'hydrogène dans le groupe $-(CH_2)_n$ pouvant éventuellement être remplacé par un groupe alkyle inférieur à 1 à 6 atomes de carbone, ou par un noyau phényle, ou deux atomes d'hydrogène géminés pouvant être remplacés par un atome d'oxygène,

$R^1$ représente un atome d'hydrogène, éventuellement remplacé par un cation métallique ou un cation amine physiologiquement tolérés, ou un reste alkyle ayant 1 à 6 atomes de carbone,

$R^2$ et $R^3$, qui peuvent être identiques ou différents, désignent un atome d'hydrogène ou de chlore, un groupe alkyle ou alcoxy ayant 1 à 4 atomes de carbone.

3. Acide N-(indéno [1,2-d] thiazol-2-yl) oxamidique, ses esters alkyliques ayant 1 à 4 atomes de carbone dans l'alkyle et ses sels avec un cation métallique ou un cation amine physiologiquement tolérés.

4. Procédé de préparation de composés de formule I, selon la revendication 1, caractérisé par le fait que l'on condense, de manière en soi connue, un aminothiazole de formule II

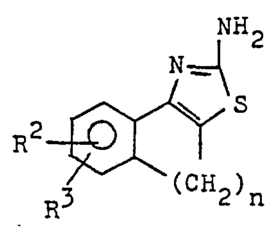

II

dans laquelle n, $R^2$, $R^3$ et $R^4$ ont les significations données plus haut, avec un halogénure d'alkyloxalyle ou un dialkyloxalate, on saponifie éventuellement le composé résultant pour obtenir l'acide et on le transforme en sel avec un cation métallique ou un cation amine physiologiquement tolérés.

5. Agent thérapeutique contenant, comme principe actif, un composé de formule I, selon la revendication 1, à côté de véhicules et diluants usuels pour des préparations pharmaceutiques.

6. Composé de formule I, selon la revendication 1, comme médicament pour la lutte contre des maladies allergiques.